# EUROPÄISCHE PATENTSCHRIFT

(11) **EP 1 052 927 B1**
(45) Veröffentlichungstag und Bekanntmachung des Hinweises auf die Patenterteilung: **05.03.2003**
(21) Anmeldenummer: 98965570.9
(22) Anmeldetag: 13.11.1998
(51) Int. Cl.: A61B 1/00

(54) **VERFAHREN UND VORRICHTUNG ZUM TRAINING VON KÖRPERTEILEN EINES MENSCHEN**
METHOD AND DEVICE FOR TRAINING BODY PARTS OF A PERSON
PROCEDE ET DISPOSITIF POUR FAIRE TRAVAILLER LES PARTIES DU CORPS D'UNE PERSONNE

(30) Priorität: 14.11.1997 DE 19750441
(43) Veröffentlichungstag der Anmeldung: 22.11.2000
(73) Patentinhaber: Hein, Achim, 91052 Erlangen (DE); Becker, Markus, 57076 Siegen (DE)
(72) Erfinder: HEIN, Achim, D-91052 Erlangen (DE); BECKER, Markus, D-57076 Siegen (DE)
(74) Vertreter: Rau, Albrecht
(86) Internationale Anmeldenummer: DE9803369
(87) Internationale Veröffentlichungsnummer: WO99025237

(56) Entgegenhaltungen:
- EP-A- 0 211 984
- WO-A-91/11218
- DE-A- 3 248 179
- DE-A- 4 021 240
- US-A- 4 633 237
- US-A- 5 375 610

## Beschreibung

Die Erfindung betrifft eine Vorrichtung zum Training von Körperteilen eines Menschen in sitzender Haltung.

Haltungsschäden werden im allgemeinen von einem Arzt festgestellt und durch entsprechendes Fachpersonal bewegungstherapeutisch behandelt. Nach einer jeweiligen Schadenserkennung wird durch Rehabilitationsmaßnahmen versucht, den Bewegungsapparat entsprechend zu stabilisieren. Es gibt jedoch schon Ansätze, entsprechende Merkmale mittels automatisierter Verfahren zu erkennen und dann zu beseitigen. Hierzu gehören insbesondere Verfahren, die aufgezeichnete Daten von Bewegungen der Arme, Beine oder des Kopfes erfassen. Die Bewegungsabläufe können dann nach der eigentlichen Bewegungstätigkeit mittels verschiedenartig realisierter Analysen evaluiert werden.

Bekannt ist auch, zur Bedienung von computergestützten Anwendungen, beispielsweise einer Textverarbeitungs-Software, als Eingabegeräte neben Tastatur und Maus fuß- oder kopfgesteuerte Eingabeeinheiten zu benutzen. Die Bemühungen gehen hier in Richtung optionaler Bedienmöglichkeiten als Ersatz für handgesteuerte Betätigungen.

Aus der DE-OS 39 39 327 ist ein Verfahren zum Verbessern einer schädlichen Körperhaltung sowie ein Meßgerät und ein Kleidungsstück zur Realisierung des erfindungsgemäßen Verfahrens beschrieben. Dabei wird der Körper durch eine asymmetrische Belastung gezwungen, eine normale Körperhaltung einzunehmen. Der Neigungswinkel von Linien, die für die Haltung charakteristische Körperteile verbinden, wird gemessen.

Aus dem deutschen Gebrauchsmuster 297 12 990 ist ein Sitzhaltungsfehler-Erfassungs- und Warnsystem bekannt, das einen Warnton ausgibt, wenn sich der Kopf eines Benutzers zu nahe an eine Oberfläche heranbewegt. Dabei wird die Abdeckung eines Lichtsensors durch den Kopf ausgewertet.

Das deutsche Gebrauchsmuster 296 12 724 beschreibt ein Gerät zur computergestützten Gleichgewichtstherapie mit einem Kugelabschnitt, der eine vergrößerte Schnittfläche als Standplattform für Personen besitzt. Eine Steuerung eines Computerprogramms erfogt mittels eingebauter Schalter. Dabei ist der Kugelabschnitt mittig in einem Schalteraufnahmering in allen seitlichen Richtungen beweglich verankert. Durch Gleichgewichtsverlagerung der Person können die sich im Schalteraufnahmering befindlichen Schalter betätigt werden. Durch den Schaltvorgang wird ein Bildschirmcursor in entsprechende Richtungen bewegt.

Die DE-OS 40 21 240 beschreibt eine Einrichtung zur Überprüfung der Körperhaltung von Personen bei der Benutzung von Trainingsgeräten, Sitzgelegenheiten oder dergleichen. Die Einrichtung weist wenigstens ein druckempfindliches Sensorelement, ein Trägerelement für das oder die Sensorelemente und eine Auswerteeinheit auf. Das Trägerelement ist so an dem jeweiligen Trainingsgerät, der Sitzgelegenheit oder dergleichen anbringbar, daß die Person in Abhängigkeit von ihrer Körperhaltung auf das oder die Sensorelemente einen bestimmten Druck ausübt. An die Auswerteeinheit sind die Ausgangssignale der Sensorelemente angelegt. Die Auswerteeinheit weist eine Anzeigeeinheit auf, die die momentan eingenommene Haltung der Person anzeigt.

Ein Gerät zum funktionellen und quantifizierten Bewerten der menschlichen Aktivität ist in der US-PS 5,375,610 beschrieben. Das Gerät umfaßt vier Hauptkomponenten: Eine Datenerfassungseinheit, eine Körperanzug-Schnittstelle, einen Körperanzug mit einer Vielzahl von Schaltern und ein graphisch orientiertes Softwarepaket. Die Schalter sind auf dem Körperanzug so angeordnet, daß sie die zu messenden Bewegungen erfassen können. Über Datenerfassungsmittel können graphische Analysemittel mit den Sensoren verbunden werden. Die graphischen Analysemittel liefern mittels Strichmännchen eine visuelle Darstellung von Körper- und Armpositionen über der Zeit in real time. Dies kann als Bewegungs-Feedback verwendet werden. Weiterhin können auf dem Bildschirm mittels Strichmännchen graphisch die verschiedenen Körperpositionen illustriert werden. Dazu wählt der Benutzer aus einer Liste die darzustellende Position aus.

Bei dem im folgenden angeführten Stand der Technik gehen die Bemühungen in Richtung optionaler Bedienmöglichkeiten für einen Rechner, die äquivalent zu handgesteuerten Bedienungen sind.

In der WO96/0144 ist ein Steuergerät für Computer oder industrielle Prozesse beschrieben. Das Konzept basiert auf dem Prinzip, einen Computer mit Bewegungen des Körpers, die über den Stuhl übertragen werden, zu steuern. Das Steuergerät ist dadurch charakterisiert, daß in einem Stuhl mehrere Wandler eingebaut sind. Ein erster Wandler ist vorgesehen, um Drehbewegungen des Stuhles zu erfassen. Er gibt Signale ab, die Bewegungen in horizontaler Richtung entsprechen. Ein weiterer Wandler erfaßt Bewegungen in vertikaler Richtung. Die Ausgangssignale der Wandler werden über ein Interface einem Sender zugeführt. Derart kann eine konventionelle Maus vollständig oder teilweise durch Bewegungen des Stuhls ersetzt werden. Drehbewegungen aktivieren z.B. einen Cursor auf einer Anzeigeeinheit in horizontaler Richtung, während Vor- und Rückwärtsbewegungen des Körpers den Cursor in vertikaler Richtung bewegen.

Im deutschen Gebrauchsmuster 297 04 478 ist eine Vorrichtung zur Betätigung eines Joysticks durch einen stehenden oder sitzenden Benutzer beschrieben. Oberhalb eines zu betätigenden Joysticks ist eine horizontal ausgerichtete Platte angeordnet, die verkippbar und beweglich gelagert ist. An ihrer Unterseite ist eine Betätigungsvorrichtung zur lateralen Bewegung des Joysticks angebracht, die bei einer Verkippung der Platte den Joystick betätigt. Ein Benutzer steht oder sitzt auf dieser Platte und verlagert sein Körpergewicht, was zu einer Verkippung der Platte, damit zur Betätigung des Joysticks und damit wiederum zu dem erwünschten Eingriff in ein Spiel oder in eine virtuelle Welt führt.

In der US-Patentschrift 4,843,538 ist ein Gerät und ein Verfahren beschrieben, mit dem über eine Abbildung eines menschlichen Körpers eine Realtime-Steuerung von Computerereignissen durchgeführt werden kann. Dabei wird eine Wahrnehmungs- und Merkmalsanalyse durchgeführt.

Der Erfindung liegt nun die Aufgabe zugrunde, eine Vorrichtung zum Training von Körperteilen eines Menschen in sitzender Haltung anzugeben, mit dem Körperbewegungen erfaßt und gesteuert werden können.

Die Aufgabe wird durch die in Anspruch 1 angegebenen Merkmale gelöst.

Damit ist es möglich, Körperbewegungen und Körperhaltungen, insbesondere auch Fehlhaltungen, anzuzeigen, zu überwachen und zu korrigieren. Die sensorische Kontrolle und die Anzeige ermöglicht eine fachkompetente Bewegungsführung. Beispielsweise wird ein zu Rehabilitationszwecken von einem Arzt für ein bestimmtes Problem erarbeitetes, spezielles Bewegungsprogramm vorgegeben. Die Kontrolle des Bewegungsprogramms erfolgt automatisch über die Sensorik, ohne daß dauerhafte Fachpersonal-Präsenz erforderlich ist.

Ein weiterer Vorteil der Erfindung besteht darin, daß im weiterhin zunehmenden Bereich der Personal Computer-Arbeitsplätze bekannte Probleme im Haltungs- und Bewegungsapparat durch den additiven oder auch optionalen Einsatz der Erfindung verbessert werden können. Der Anwender kann dauerhaft oder zeitlich begrenzt beispielsweise mittels einer Rückenentspannungs-Software zum Gebrauch des bewegungsaktivierenden Eingabesystems aufgefordert werden. Diese Möglichkeit würde der Bewegungsverarmung und Rückenverkrümmung in diesem Arbeitsbereich prophylaktisch entgegenwirken.

Weiterhin besteht die Möglichkeit, virtuelle Rehabilitationswelten aufzubauen, bei denen z.B. ein Arzt oder Therapeut einer Therapiegruppe eine Bewegung vorgibt. Das Protokoll der Bewegung kann zusätzlich dem Arzt oder Therapeuten übermittelt werden. Zur Kontrolle können die Teilnehmer der Therapiegruppe die Darstellung der Bewegung auf einem Monitor wie in einem Spiegel kontrollieren.

Eine vorteilhafte Ausgestaltung zeichnet sich dadurch aus, daß die Sollbewegung als ein Sollweg vorgegeben wird, den eine Marke auf dem Monitor zurücklegen soll. Die Vorgabe eines Sollwegs ermöglicht eine gezielte therapeutische Bewegungsführung.

Bei einer weiteren vorteilhaften Ausgestaltung de Erfindung wird die Bewegung des Menschen über ein Sitzgerät auf die Sensoreinheit übertragen. Damit können verschiedene unter therapeutischen Gesichtspunkten entwickelte Sitzgeräte verwendet werden.

Eine weitere vorteilhafte Ausgestaltung zeichnet sich dadurch aus, daß aus der Sollbewegung und den Bewegungssignalen ein Qualitätssignal gebildet wird als Maß der Übereinstimmung von Sollbewegung und Bewegung. Das Qualitätssignal gibt dem Patienten eine Rückkopplung über den Gesamterfolg der gerade durchgeführten therapeutischen Bewegungen. Es wird somit ein therapeutisches Qualitätsmaß eingeführt, daß der Vergleichbarkeit verschiedener Personen oder auch verschiedener Trainingseinheiten einer Person dienen kann.

Vorzugsweise werden die Bewegungssignale quasikontinuierlich erfaßt und dargestellt. Quasi-kontinuierlich heißt hier, daß bei einer digitalen Signalverarbeitung die Abtastrate zur Umwandlung der in ein digitales Format der im allgemeinen zunächst in analoger Form vorliegenden Bewegungssignale so hoch ist, daß ein Betrachter den Eindruck einer kontinuierlichen Erfassung und Darstellung hat.

Die Auflösung der Sensorelemente entspricht der Auflösung eines Monitors von z.B. ca. 1200x800 Pixeln. Die Bewegungssignale können nach einer Aufbereitung in analoger Form oder auch in digitaler Form der Verarbeitungseinheit zugeführt werden.

Eine vorteilhafte Ausgestaltung zeichnet sich dadurch aus, daß mit der Sensoranordnung ein Sitzgerät verbunden ist zur Übertragung von durch die Bewegung des sitzenden Menschen erzeugte Kräfte auf die Sensoranordnung. Damit kann bei der Anwendung eine normale Grundhaltung eingenommen werden.

Das Sitzgerät kann als Stuhl oder Hocker ausgestaltet sein. Bei einer besonders vorteilhaften Ausgestaltung ist als Sitzgerät ein Sitzball verwendet. Sitzbälle sind medizinisch anerkannt zur Therapie von Haltungsschäden und transformieren zudem die Körperbewegung in leicht zu detektierende Rollbewegungen.

Bei einer weiteren vorteilhaften Ausgestaltung umfaßt die Sensoranordnung eine Vielzahl von in einer Fläche angeordneten Sensorelementen. Damit kann die Bewegung eines darauf liegenden Sitzballs durch Verlagerung des Abdrucks des Sitzballs auf der Sensoranordnung erfaßt werden. Die Sensorelemente detektieren die Bewegung beispielsweise drucksensitiv, abstands- oder wegsensitiv, drehsensitiv, geschwindigkeitssensitiv oder beschleunigungssensitiv. Die Sensoranordnung kann z.B. als entsprechend sensitive Matte ausgestaltet sein. Als Sensorprinzipien können elektrische, magnetische, optische oder auch mechanische Rezeptoren, wie z.B. Lichtschranken, Piezosensoren, Hallsensoren usw. verwendet werden. Weitere Ausgestaltungen der Erfindung sind durch die Unteransprüche gekennzeichnet.
Ausführungsbeispiele der Erfindung werden im folgenden anhand von neun Figuren erläutert. Es zeigen:
- Figur 1: in einer Übersichtsdarstellung die wesentlichen Einheiten einer Vorrichtung zum Training von Körperteilen eines Menschen in sitzender Haltung,
- Figur 2: eine erste Vorrichtung zum Training von Körperteilen mit einem Lichtgitternetz als Sensoreinheit,
- Figur 3: eine zweite Vorrichtung zum Training von Körperteilen mit einer elektrischen Kontaktmatrix als Sensoreinheit,

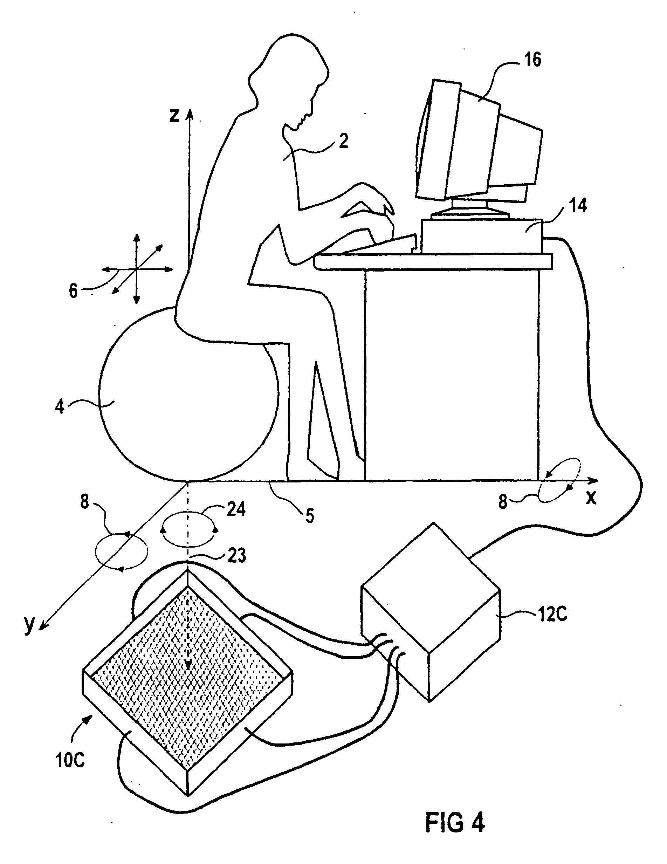

- Figur 4: eine dritte Vorrichtung zum Training eines Körperteils mit einer Druck-Kontaktmatrix als Sensoreinheit,

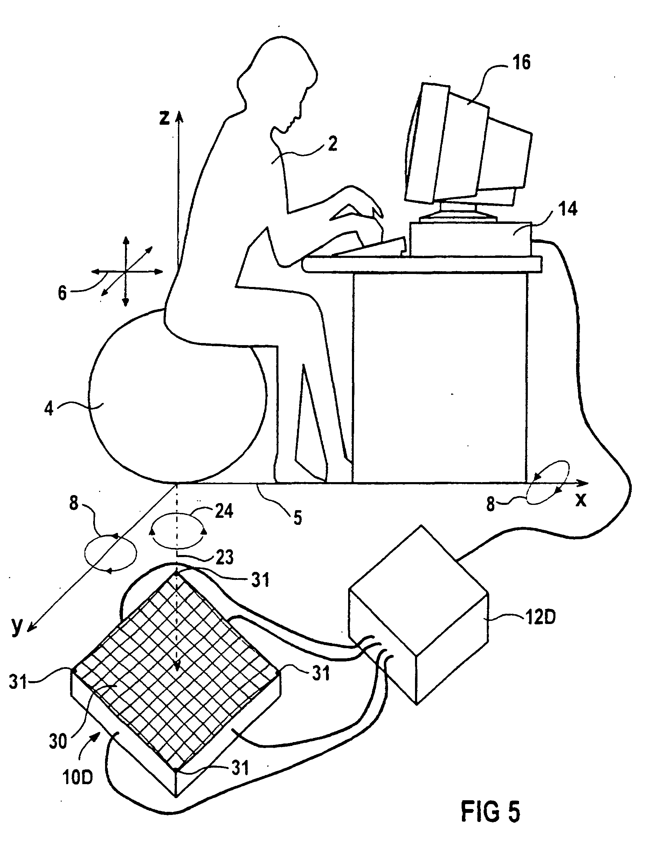

- Figur 5: eine vierte Vorrichtung zum Training eines Körperteils mit einem an mindestens drei Befestigungspunkten befestigten Netz in der sensorischen Konzeption,
- Figur 6: eine fünfte Vorrichtung zum Training von Körperteilen mit einer beweglich gelagerten Platte innerhalb der sensorischen Konzeption,

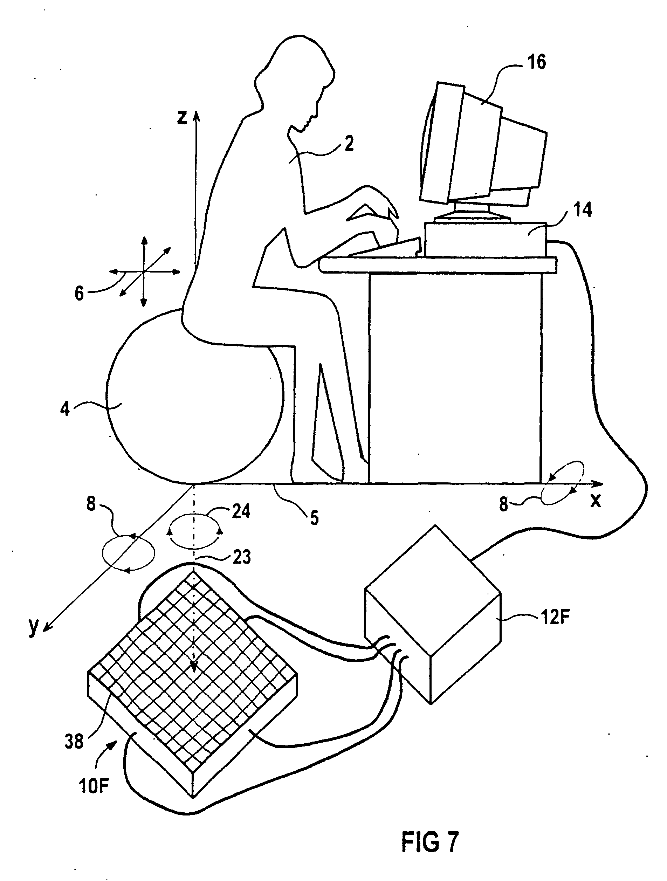

- Figur 7: eine sechste Vorrichtung zum Training von Körperteilen mit einer sensitiven Matte als Sensoreinheit,

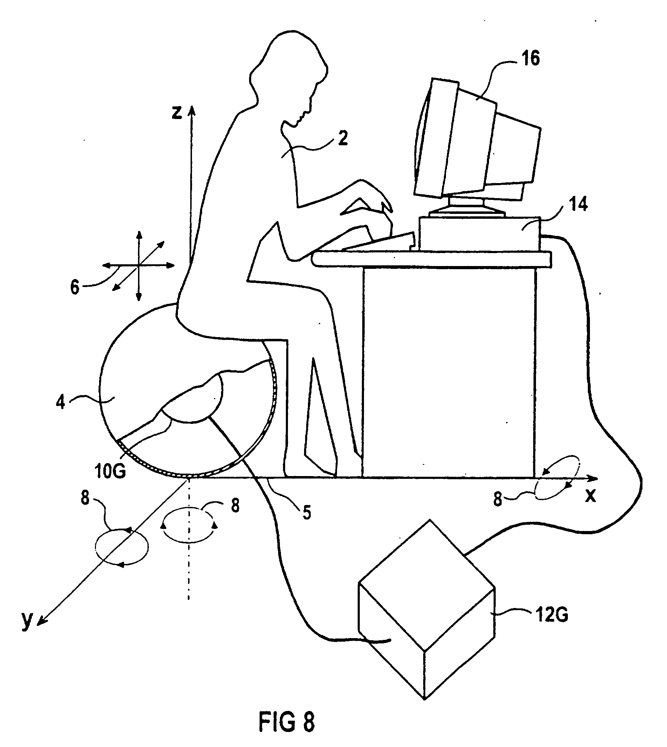

- Figur 8: eine siebte Vorrichtung zum Training von Körperteilen mit einem Gyroskop als Sensoreinheit,
- Figur 9: eine achte Vorrichtung mit einer drahtlosen Übertragung der Bewegungssignale und
- Figur 10: eine Darstellung eines Monitorbildes mit visuellen Vorgaben von Sollbewegungen.

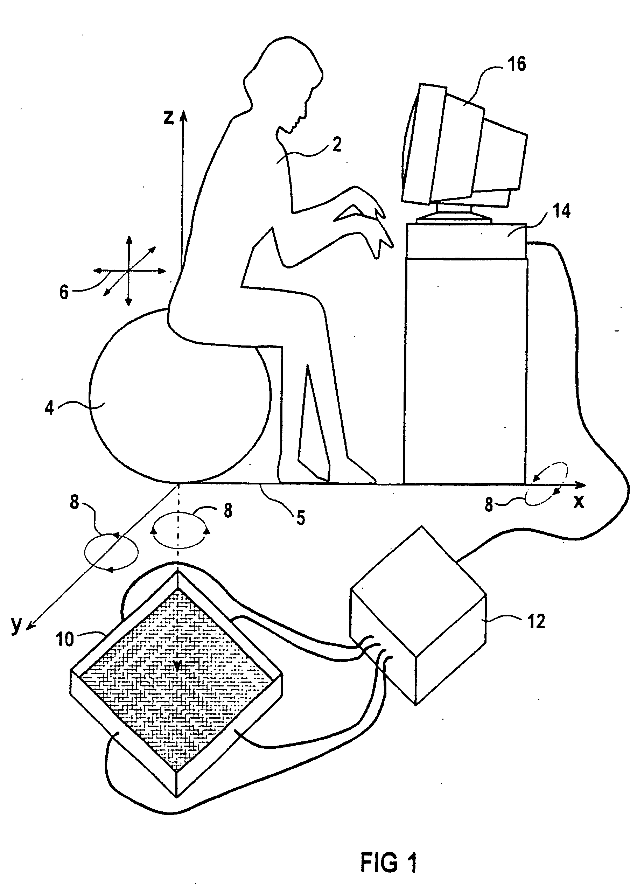

Figur 1 zeigt die prinzipielle Anordnung der Vorrichtung zum Training von Körperteilen eines Menschen oder einer Person 2 in sitzender Haltung. Die Person 2 sitzt auf einem herkömmlichen medizinischen Sitzball 4, wobei sie sich mit den Füßen auf dem Boden abgestützt. Der Ursprung eines räumlichen Koordinatensystems 5 liegt im Berührungspunkt des Sitzballs 4 mit seiner Auflagefläche, die hier im wesentlichen der von den Koordinaten x und y aufgespannten Fläche entspricht. Die Person 2 kann auf dem Sitzball 4 Bewegungen in allen drei Raumkoordinatenrichtungen x, y und z ausführen, verdeutlicht durch ein Pfeilkreuz 6. Diese Bewegungen 6 werden, außer der vertikalen Bewegung in z-Richtung, vom Sitzball 4 in Rollbewegungen umgesetzt, die sich als Drehbewegungen um die x-, y- und z-Achse darstellen lassen. Dies ist durch Kreise mit Pfeilen 8 symbolisiert. Die Drehbewegungen 8 bewirken z.B. Translationsbewegungen, wenn als Sitzgerät der Sitzball 4 verwendet wird. Die Ortsveränderung des Sitzballs 4 wird von einer Sensoranordnung 10 mit hoher Genauigkeit und Ortsauflösung, z. B. entsprechend der Auflösung eines Monitors mit ca. 1200x800, erfaßt.

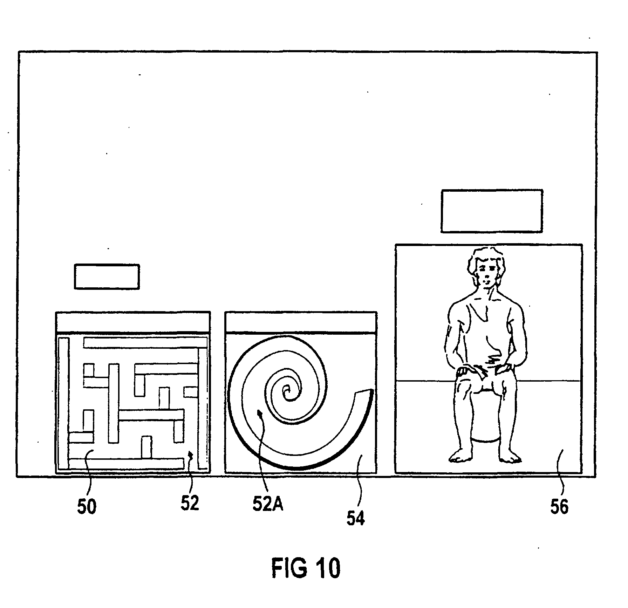

Die Sensoranordnung 10 ist mit einer Auswerteeinheit 12 verbunden, die die von den Bewegungen ausgelösten Sensorantworten aufbereitet und als digitalisierte oder auch analoge Signale ausgibt. Gegebenenfalls umfaßt die Auswerteeinheit 12 auch eine Stromversorgung für die Sensoranordnung 10. Die Auswerteeinheit 12 ist mit einem Rechner 14 verbunden, der auf der Basis der Bewegungssignale einen Cursor auf einem Monitor 16 steuert. Gleichzeitig mit dem Cursor wird auf dem Monitor 16 ein Weg für den Cursor vorgegeben. Die Person 2 muß nun in sitzender Haltung durch Bewegungen auf dem Sitzball den Cursor entsprechend der Wegvorgabe bewegen, wie nachfolgend noch anhand von Figur 10 beschrieben ist.

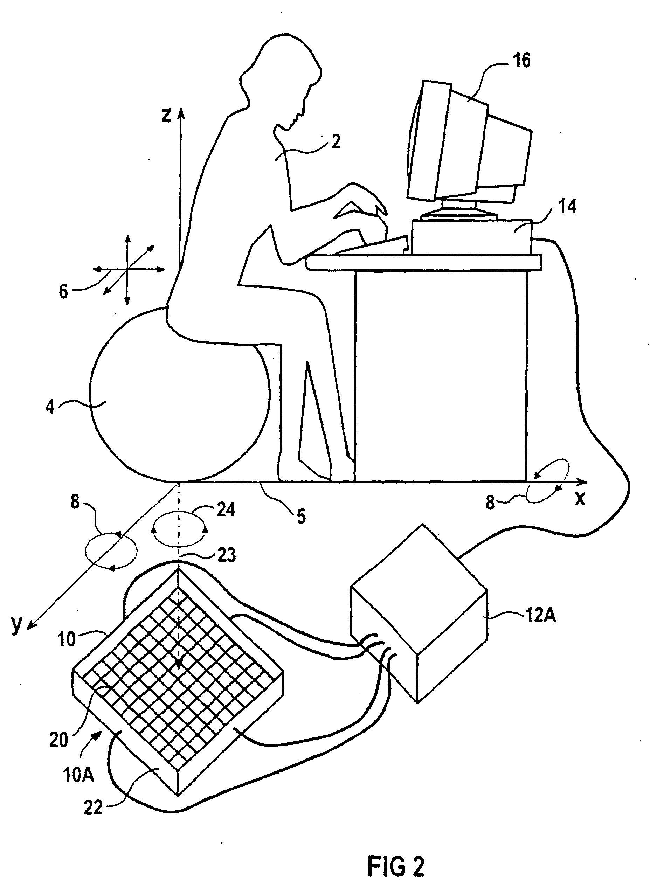

Eine erste praktische Realisierung des Sensorkonzepts zeigt Figur 2 in einer teilweisen Explosionsdarstellung. Eine Sensoranordnung 10A umfaßt ein Lichtgitternetz 20, das durch Anbringen entsprechender Lichtschranken an Außenwänden 22 der Sensoranordnung 10A erzeugt wird. Der Sitzball wird innerhalb des Lichtgitternetztes 20 bewegt, was durch einen Pfeil 23 veranschaulicht ist. Mehrdimensionale bewegungs- und gewichtsverursachte Positionen und/oder Positionsveränderungen des Sitzgeräts 4 führen zu Abschattungen der entsprechenden Lichtschranken. Die damit erzeugten Signale werden mit einer entsprechenden Auswerteeinheit 12A in ein digitales Protokollformat umgewandelt. Die Verdrehung des Sitzgeräts 4 um die z-Achse wird mit einem Drehsensor, symbolisiert durch einen Kreis 24 mit Pfeilspitzen, erfaßt und ebenfalls über die Auswerteeinheit 12A dem Rechner 14 zugeführt. Die Person 2 betätigt hier zusätzlich eine Eingabetastatur während des Bewegungstrainings.

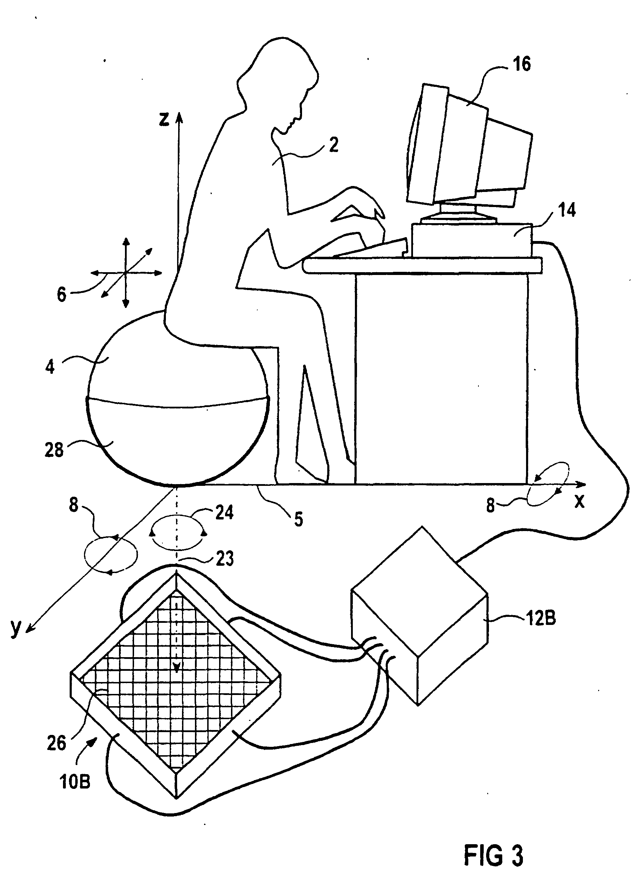

Bei der in Figur 3 dargestellten Konzeption wird eine elektrische Kontaktmatrix zur Positions- und Bewegungserkennung des Sitzgeräts 4 verwendet. Eine damit realisierte Sensoranordnung 10B umfaßt ein elektrisches Kontaktraster 26. Die im Berührungsbereich mit dem Kontaktraster 26 liegende Oberfläche des Sitzgeräts 4 ist mit einer elektrisch leitfähigen Schicht 28 belegt. Durch Kontaktschluß (Pfeil 23) der leitfähigen Schicht 28 mit dem Kontaktraster 26 werden Bewegungsund Ortssignale erzeugt, die von der Auswerteeinheit 12B wiederum in ein digitales Protokollformat umgewandelt werden. Rotationen des Sitzballs 4 um die vertikale z-Achse werden ebenfalls wie bei der vorangegangenen Ausführung mit einem Drehsensor, z.B. einem Gyroskop, erfaßt und dem Rechner in digitaler Form zugeleitet.

Ähnlich wie bei der voranstehend beschriebenen Sensorkonzeption wird bei der in Bild 4 dargestellten Sensorkonzeption eine Sensoranordnung 10C mit einer Kontaktmatrix verwendet, um die mehrdimensionale Position und/oder Positionsänderung des Sitzgeräts 4 zu detektieren. Die Kontaktmatrix ist hier als Druck-Kontaktmatrix ausgeführt, die bei einwirkender Druckkraft entsprechende Kontakte schließt. Mittels der Auswerteeinheit 12C wird ein entsprechendes Protokollformat berechnet.

Die in Bild 5 dargestellte Konzeption benutzt eine Sensoranordnung 10D mit einem Netz 30, das an mindestens drei Befestigungspunkten 31 - hier an den vier Eckpunkten der Sensoranordnung 10D - befestigt ist. An den Befestigungspunkten 31 sind Weg- und/oder Drucksensoren angeordnet, die die mehrdimensionale bewegungs- und gewichtsverursachte Position und/oder Positionsveränderung des darauf gelagerten Sitzgeräts 4 unter Zuhilfenahme einer entsprechenden Auswerteeinheit 12D in ein für den Rechner 14 lesbares Protokollformat umwandelt. Auch hier ist zusätzlich zur Erfassung der Drehbewegung um die z-Achse ein Drehsensor 24 vorgesehen.

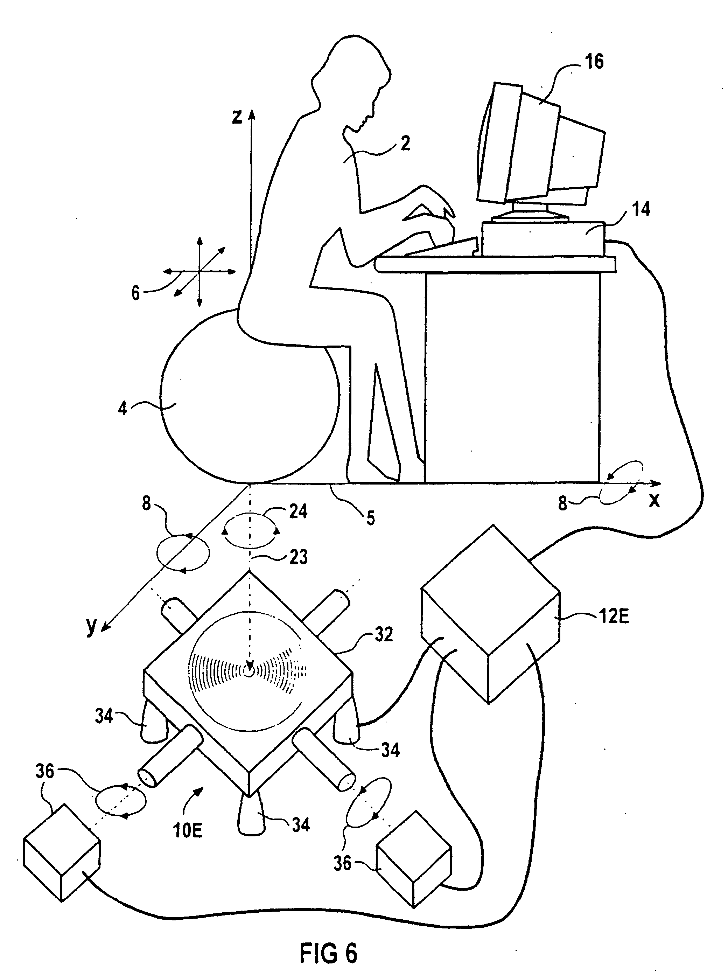

Bei der in Figur 6 gezeigten Sensorkonzeption umfaßt eine Sensoranordnung 10E eine starre Platte 32, die an mindestens drei Punkten 34 federnd abgestützt ist. Die als Auflagefläche für das Sitzgerät 4 dienende Plattenoberfläche kann eben oder auch konkav ausgebildet sein. Die konkave Ausgestaltung hat den Vorteil, daß der Bewegung zum Rand stabilisiert wird. Kippbewegungen der Platte werden mit zwei Drehsensoren 36 erfaßt. Zusätzlich ist zur Erfassung der Drehbewegung des Sitzgeräts 4 um die senkrechte z-Achse der Drehsensor 24 vorgesehen. Die Drehbewegungen werden von der Auswerteeinheit 12E in ein entsprechendes digitales und rechnerkompatibles Format umgewandelt.

Bild 7 zeigt als eine Sensoranordnung 10F mit einer Sensormatte, die Bewegungsänderungen mittels einer entsprechenden sensitiven Matte 38 akquiriert. Die von der Matte 38 abgegebenen Signale werden werden von einer Auswerteeinheit 12F in ein rechnerkompatibles Format umgewandelt.

Die in Bild 8 skizzierte Ausführung der Vorrichtung zum Training von Körperteilen erfaßt Bewegungen und/oder Bewegungsänderungen des Sitzballs 4 mittels einer im oder am Ball angebrachten Sensoranordnung 10G. Hier ist die Sensoranordnung 10G innerhalb des Sitzballs 2 befestigt, wobei der Sitzball 4 teilweise aufgebrochen dargestellt ist. Die Sensoranordnung 10G nutzt die bei Bewegungen und Bewegungsänderungen entstehenden Coriolis-Kräfte und/oder relativistische Laufzeiteffekte von Licht, das durch entsprechend angeordnete Glasfaserleitungen geführt ist. Auch die Abhängigkeit des Sagnak-Effekts von Bewegungen und/oder Bewegungsänderungenkönnen zur Messung der Bewegung genutzt werden. Beispielsweise kommen modifizierte Schwinggabelsensoren oder modifizierte Gyroskope zum Einsatz. Die Neigung und/oder Verdrehung des Sitzgeräts 4 im Raum beeinflußt die obengenannten Effekte und erzeugt entsprechende Signale, die mittels einer Auswerteeinrichtung 12G in bewegungsproportionale Signale umgewandelt werden.

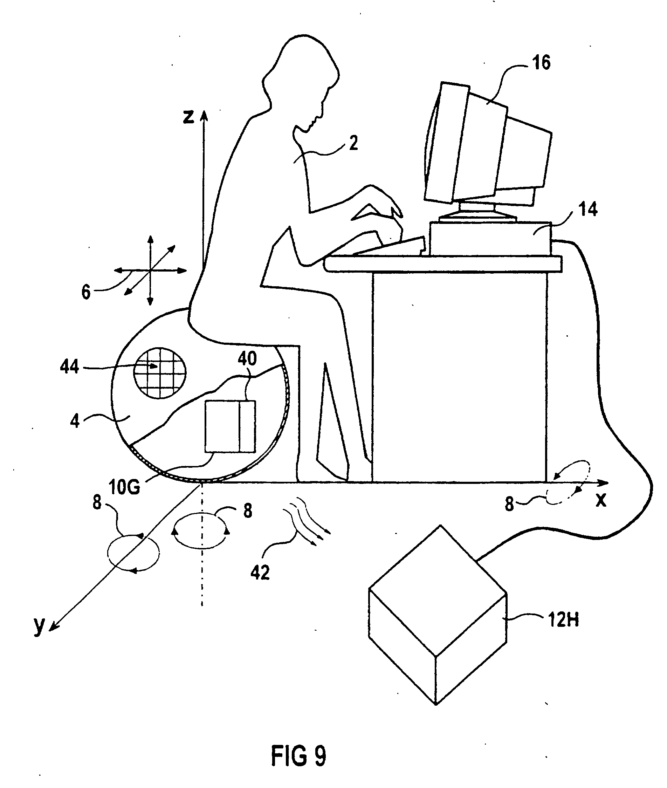

Das in Figur 9 skizzierte Sensorkonzept verwendet ebenfalls am oder im Sitzball angeordnete Sensoren 10G, die jedoch mit einem Sender 40 zur drahtlosen Signalübertragung 42 der Sensorantworten an eine Auswerteeinheit 12H verbunden ist. Die Stromversorgung kann z.B. über Solarzellen 44 erfolgen, die an einer äußeren Oberfläche des Sitzgeräts 4 angeordnet sind.

Figur 10 zeigt eine Graphik, die vom Rechner 14 erzeugt und auf dem Monitor 16 dargestellt wird. Die Graphik umfaßt eine erste Bewegungsvorgabe 50 in Form eines Labyrinths. Ein gleichzeitig mit der Bewegungsvorgabe 50 dargestellter Cursor oder eine bewegliche Sichtmarke 52 soll von der zu therapierenden Person 2 durch entsprechende Bewegungen auf dem Sitzgerät 4 durch das Labyrinth 50 geführt werden. Die Bewegungen des Sitzgeräts 4 werden von der Sensoreinheit 10 erfaßt und von dem Rechner 14 in entsprechende Steuersignale für den Cursor 52 umgewandelt. Wird der Cursor 52 fehlerfrei, z. B. ohne die den Weg begrenzenden Seitenwände zu berühren, zum Ziel geführt, erscheint ein Erfogssignal auf dem Monitor 16. Der Person 2, die das Bewegungstraining durchführen soll, wird eine alternative zweite Bewegungsvorgabe 54 in Form einer Spirale angeboten. Hier soll ein Cursor 52A im vorgegebenen Wegbereich zum Mittelpunkt geführt werden. Im Gegensatz zum Labyrinth 50 stehen bei dieser Bewegungsvorgabe 54 kreisende Bewegungen im Mittelpunkt des Trainings.

Wenn die trainierende Person 2 auch noch mit einer Videokamera aufgenommen wird, dann kann zusätzlich zur Bewegungsvorgabe 50 oder 54 ein Einblendbild 56 mit einer entsprechenden Darstellung auf eine Aktivierung hin auf dem Monitor 16 wiedergegeben werden.

## Patentansprüche

1. Vorrichtung zum Training von Körperteilen eines Menschen (2) in sitzender Haltung, umfassend:
a) ein von einem Boden abgestütztes Sitzgerät (4), wobei das Sitzgerät (4) eine Sitzfläche für einen Menschen (2) aufweist, um darauf zu sitzen;
b) einen Monitor(16), um dem Menschen (2) eine durchzuführende Sollbewegung anzuzeigen;
c) eine Sensoranordnung (10) zum Erzeugen von Bewegungssignalen aus einer Bewegung des Menschen (2) auf der Sitzfläche, wobei die Sensoranordnung (1) mit dem Sitzgerät (4) verbunden ist; und
d) eine mit der Sensoranordnung (10) verbundene Verarbeitungseinheit (12) zum Erzeugen einer Darstellung der Bewegung aus den Bewegungssignalen, wobei die Darstellung der Bewegung durch den Monitor (16) angezeigt ist.

2. Vorrichtung nach Anspruch 1, **dadurch gekennzeichnet, daß** das Sitzgerät (4) als Sitzball ausgebildet ist.

3. Vorrichtung nach Anspruch 1 oder 2, **dadurch gekennzeichnet, daß** die Sensoranordnung (10) eine Vielzahl von in einer Fläche angeordneten Sensorelementen umfaßt.

4. Vorrichtung nach Anspruch 1 oder 2, **dadurch gekennzeichnet, daß** die Sensoranordnung (10E) ein plattenförmiges starres Element (32) umfaßt, daß das Element (32) in einer Verkippmechanik gelagert ist und daß die Verkippmechanik mindestens zwei Drehsensoren (36) umfaßt.

5. Vorrichtung nach Anspruch 4, **dadurch gekennzeichnet, daß** das plattenförmige Element (32) eine konkave Oberfläche besitzt.

6. Vorrichtung nach Anspruch 1 oder 2, **dadurch gekennzeichnet, daß** die Sensoranordnung (10D) ein netzartiges Element (30) umfaßt, das randseitig an mindestens drei Befestigungspunkten (31) befestigt ist und daß an den Befestigungspunkten Sensoren angeordnet sind.

7. Vorrichtung nach einem der Ansprüche 1 bis 6, **dadurch gekennzeichnet, daß** das Sitzgerät (4) mit mindestens einem Gyroskop (10G) verbunden ist zur Erfassung einer Drehbewegung des Sitzgeräts (4).

8. Vorrichtung nach Anspruch 7, **dadurch gekennzeichnet, daß** das Gyroskop (10G) mit einem Sender (40) zur drahtlosen Übertragung der Drehbewegungssignale verbunden ist.

9. Vorrichtung nach einem der Ansprüche 1 bis 8, **dadurch gekennzeichnet, daß** das Sitzgerät eine elektrische Energiequelle (44) umfaßt.

## Claims

1. Device for training body parts of a person (2) in a sitting position, comprising:
a) a seat device (4) supported by a ground, wherein the seat device (4) has a seat for a person (2) to sit on it;
b) a monitor (16) for showing the person (2) a specified movement to be performed;
c) a sensor arrangement (10) for generating movement signals from a movement of the person (2) on the seat, wherein the sensor arrangement (10) is connected to the seat device (4); and
d) a processing unit (12) connected to the sensor arrangement (10) for generating a representation of the movement from the movement signals, wherein the representation of the movement is displayed by the monitor (16).

2. Device according to claim 1, **characterized in that** the seat device (4) is formed as a seat ball.

3. Device according to claim 1 or 2, **characterized in that** the sensor arrangement (10) comprises a multitude of sensor elements which are arranged in a plane.

4. Device according to claim 1 or 2, **characterized in that** the sensor arrangement (10E) comprises a plate-shaped rigid element (32), that the element (32) is run on bearings in a tilt mechanism and that the tilt mechanism comprises at least two rotation sensors (36).

5. Device according to claim 4, **characterized in that** the plate-shaped element (32) has a concave surface.

6. Device according to claim 1 or 2, **characterized in that** the sensor arrangement (10D) comprises a net-like element (30) which is secured along its edges in at least three fastening points (31) and that sensors are provided at the fastening points.

7. Device according to one of claims 1 to 6, **characterized in that** the seat device (4) is connected to at least one gyroscope (10G) for detecting a rotary movement of the seat device (4).

8. Device according to claim 7, **characterized in that** the gyroscope (10G) is connected to a transmitter (40) for a wireless transmission of the rotary movement signals.

9. Device according to one of claims 1 to 8, **characterized in that** the seat device comprises an electric energy source (44).

## Revendications

1. Dispositif pour l'entraînement de parties du corps d'un homme (2) dans une position assise, comportant:
a) un dispositif de siège (4) supporté par un plancher, le dispositif de siège (4) ayant une surface de siège pour un homme (2) pour s'installer là-dessus;
b) un moniteur (16) pour communiquer à l'homme (2) un mouvement exigé qui est à faire;
c) un bloc détecteur (10) pour produire des signaux de mouvement à partir d'un mouvement de l'homme (2) sur la surface de siège, le bloc détecteur (10) étant relié au dispositif de siège (4); et
d) une unité de traitement (12) reliée au bloc détecteur (10) pour produire une illustration du mouvement à partir des signaux de mouvement, l'illustration du mouvement étant communiquée par le moniteur (16).

2. Dispositif selon la revendication 1, **caractérisé en ce que** le dispositif de siège (4) est un ballon de siège.

3. Dispositif selon la revendication 1 ou 2, **caractérisé en ce que** le bloc détecteur (10) comporte une multiplicité d'éléments détecteurs arrangés dans une plaine.

4. Dispositif selon la revendication 1 ou 2, **caractérisé en ce que** le bloc détecteur (10E) comporte un élément (32) rigide en forme de plaque, **en ce que** l'élément (32) est logé dans un mécanisme de pivotement, et **en ce que** le mécanisme de pivotement comprend au moins deux détecteurs de rotation.

5. Dispositif selon la revendication 4, **caractérisé en ce que** l'élément (32) en forme de plaque a une surface concave.

6. Dispositif selon la revendication 1 ou 2, **caractérisé en ce que** le bloc détecteur (10D) comporte un élément réticulé (30) dont le bord est fixé à au moins trois points de fixation (31), et **en ce que** des détecteurs sont disposés aux points de fixation.

7. Dispositif selon l'une quelconque des revendications 1 à 6, **caractérisé en ce que** le dispositif de siège (4) est relié à au moins un gyroscope (10G) pour la détection d'un mouvement de rotation du dispositif de siège (4).

8. Dispositif selon la revendication 7, **caractérisé en ce que** le gyroscope (10G) est relié à un émetteur (40) pour la transmission sans-fil des signaux de mouvement de rotation.

9. Dispositif selon l'une quelconque des revendications 1 à 8, **caractérisé en ce que** le dispositif de siège comporte une source d'énergie électrique (44).
